# EUROPÄISCHE PATENTANMELDUNG

(11) **EP 2 082 748 A1**
(43) Veröffentlichungstag der Anmeldung: **29.07.2009**
(21) Anmeldenummer: 09004226.8
(22) Anmeldetag: 21.07.2003
(51) Int. Cl.: A61K 38/36, A61K 38/37, A61K 38/48

(54) **Thrombingenerierfähige und thrombinhältige pharmazeutische Wirkstoffzubereitungen und Arzneimittel**

(30) Priorität: 23.07.2002 AT 11132002
(62) Teilanmeldung aus: 03770983.9
(71) Anmelder: Bio & Bio Licensing SA, 1724 Luxembourg (LU)
(72) Erfinder: Eibl, Johann, Dr., 1180 Wien (AT)
(74) Vertreter: Schwarz, Albin

(57) **Zusammenfassung**

Die Erfindung betrifft eine pharmazeutische Wirkstoffzubereitung zur Herstellung eines thrombingenerierfähigen oder thrombinhältigen Arzneimittels und ist **dadurch gekennzeichnet, dass** sie enthält: (A) aus Plasma oder gentechnologisch gewonnenes Prothrombin (Gerinnungsfaktor 11), (B) aus Plasma oder gentechnologisch gewonnene Gerinnungsfaktoren V, VIII, IX, X, die zumindest teilweise in aktiviertem Zustand vorliegen können, und aus Plasma oder gentechnologisch gewonnenen Gerinnungsfaktor Xla, und (C) prionensichere, gerinnungsaktive Phospholipide, wobei die Phospholipide gegebenenfalls in Liposomen enthalten sind.

## Beschreibung

Die Erfindung betrifft eine thrombingenerierfähige und thrombinhältige pharmazeutische Wirkstoffzubereitung und daraus hergestellte Arzneimittel.

### Einteilung

Werden Blutgefäße verletzt, so tritt Blut aus dem vaskulären Raum aus und gerinnt. Die verletzten Blutgefäße werden durch das gerinnende Blut verschlossen und schützen so den Organismus vor großen Blutverlusten. Die Blutgerinnung wird durch das Enzym Thrombin verursacht, das sich aus seinem Zymogen, Prothrombin, generiert und die Umwandlung des im Blutplasma vorhandenen Proteins Fibrinogen in unlösliches Fibrin bewirkt. Das austretende Blut gerinnt innerhalb von Minuten, wobei nur ein Sechstel des im Plasma enthaltenen Fibrinogens in Fibrin umgesetzt wird. In dem noch stark fibrinogenhältigen geronnenen Blut setzt sich die Thrombinbildung weiter fort, so dass es schließlich zu einer kompletten Umwandlung des gesamten, im geronnenen Blut noch vorhandenen Fibrinogens in Fibrin kommt. Dieser Vorgang der fortgesetzten Thrombinbildung und Gerinnung nimmt längere Zeit in Anspruch und kann auch erst nach mehreren Stunden vollständig beendet sein.

Bei der Umwandlung von Fibrinogen in Fibrin bewirkt Thrombin zunächst die Abspaltung bzw. teilweise Abspaltung der Fibrinopeptide A und B von den beiden Enden des Fibrinogenmoleküls, wodurch zunächst noch lösliche Fibrinmonomere entstehen. Diese können lateral zu Fibrillen aggregieren, die sich dann zu einem Fibrinnetzwerk ausbilden (Blombäck). Dieses im geronnenen Blut entstandene Fibrinnetzwerk haftet am verletzten Gewebe bzw. dem Wundbett an und führt so zur Blutstillung und zum Wundverschluss.

Thrombin bewirkt auch die Überführung des Gerinnungsfaktors XIII, des Zymogens des Gerinnungsfaktors XIIIa, in eine Transglutaminase. Diese vernetzt Proteine wie Fibrin, Fibrinmonomere, Fibrinogen und andere im Blutplasma vorkommende Proteine durch kovalente Bindungen. Insbesondere die Quervernetzung von Fibrin ist für die Stabilität des sich ausbildenden Fibrinnetzwerkes von großer Bedeutung (Siebenlist et al.).

Thrombin führt auch den thrombinaktivierbaren Fibrinolyseinhibitor (TAFI) in eine Karboxypeptidase (TAFIa) über, die von Fibrin karboxyterminale Lysinreste abspaltet und dadurch die Bildung des Tissue-Plasminogen-Aktivator-Plasminogen-Fibrin-Komplexes unterbindet, der für die Überführung von Plasminogen in Plasmin erforderlich ist (Booth).

*In vitro* wird die Bildung von Thrombin im Blut oder Blutplasma und damit das Einsetzen des Gerinnungsvorganges durch Gewebsextrakte ermöglicht. Solche Extrakte können am besten aus Gehirn mit wässrigen Medien oder auch mit organischen Lösungsmitteln erhalten werden (Morawitz).

Das mit geeigneter Pufferlösung extrahierbare, als Thromboplastin bezeichnete gerinnungsaktive Material besteht aus einem Apoprotein, dem Tissue Faktor, und gerinnungsaktiven Lipiden. Schon geringe Mengen Thromboplastin, wenn sie Blut oder Plasma zugesetzt werden, führen zu einer schnellen Gerinnung.

Der mit organischen Lösungsmitteln extrahierbare gerinnungsaktive Bestandteil des Gehirns wird als partielles Thromboplastin bezeichnet und besteht im wesentlichen aus gerinnungsaktiven Lipiden. Bei Zusatz zu Blut oder Blutplasma kommt es nur dann zu einer schnellen Gerinnung, wenn noch aktivierende Substanzen, wie z.B. Kaolin oder Glaspulver, zugesetzt werden.

Daraus entstand die Vorstellung einer Aktivierungskaskade von Enzymen, die letztlich in der Entstehung des Enzyms Thrombin endet, das die Blutgerinnung veranlasst (Davie et al.; MacFarlane)

Der durch Thromboplastin ausgelöste Gerinnungsvorgang wird als *extrinsic* Pathway der Blutgerinnung bezeichnet, der durch das partielle Thromboplastin bewirkte Gerinnungsvorgang als *intrinsic* Pathway. Beide Vorgänge haben gemeinsam, dass sie, wenn auch auf unterschiedlichem Weg, den Gerinnungsfaktor X in Xa umsetzen. Demnach wird bei dem ersten Teil des Gerinnungsvorganges, der zur Aktivierung des Gerinnungsfaktors X führt, zwischen dem *extrinsic* und *intrinsic* Tenase-Pathway unterschieden. In einem weiteren Enzymkomplex, der Prothrombinase, setzt der Gerinnungsfaktor Xa Prothrombin in Thrombin um. Dieser Vorgang wird als *common* Pathway bezeichnet. Das Enzymsystem, das mit Hilfe von Thromboplastin den Gerinnungsfaktor Xa generiert, wird als *extrinsic* Tenasekomplex bezeichnet, im Gegensatz zu dem Enzymsystem, in dem das partielle Thromboplastin eine Rolle spielt, dem *intrinsic* Tenasekomplex.

Es ist die vorherrschende Ansicht, dass der Tissue Faktor gemeinsam mit dem Gerinnungsfaktor VIIa der nach Verletzungen unter Mitwirkung der Blutplättchen die Blutgerinnung auslöst (Rapaport et al.). Tissue Faktor kommt in fast allen Geweben in stark unterschiedlichen Mengen gemeinsam mit gerinnungsaktiven Lipiden vor und beide Substanzen bilden, wenn sie mit Blut in Berührung kommen, den *extrinsic* Tenasekomplex, da im Blut ständig geringe Mengen des aktivierten Gerinnungsfaktors VII vorhanden sind (Drake et al.). Der *extrinisic* Tenasekomplex führt sowohl den Gerinnungsfaktor X in Xa über als auch den Gerinnungsfaktor IX in IXa, der weiters X in Xa umwandelt. Die Aktivierung von Faktor X und die in weiterer Folge resultierende Bildung von Thrombin kommt aber durch den Tissue-Faktor-Pathway-Inhibitor (TFPI) sehr rasch zum Stillstand. Der temporär gebildete extrinsic Tenasekomplex sowie der Gerinnungsfaktor XIa führen unabhängig voneinander zur Aktivierung des *intrinsic* Tenasekomplexes, der in bezug auf Aktivierung des Gerinnungsfaktors X etwa 50fach aktiver ist als der *extrinsic* Tenasekomplex. Der *intrinsic* Tenasekomplex setzt sich aus den aktivierten Gerinnungsfaktoren IXa, VIIIa und gerinnungsaktiven Phospholipiden zusammen und wird nicht durch TFPI gehemmt (von dem Borne et al.). Das Enzym Gerinnungsfaktor IXa, das den Gerinnungsfaktor X in Xa überführt, ist im *intrinsic* Tenase-Komplex in seiner Wirkung um das 100.000 bis 1,000.000flache gesteigert. Diese Steigerung erfolgt durch den Kofaktor VIIIa, der selbst kein Enzym ist, und bestimmte Phospholipide bei optimaler Ca-lonenkonzentration.

Da der *intrinsic* Tenasekomplex sehr schnell Gerinnungsfaktor X in Xa überführt und dieser mit dem Kofaktor Va und gerinnungsaktiven Phospholipiden Prothrombin aktiviert, kommt es zu einer äußerst raschen Bildung großer Mengen Thrombins (Mann et al.).

Die einzelnen Gerinnungsfaktoren und Blutplättchen - die wesentlichen, für den Gerinnungsvorgang verantwortlichen Komponenten im Blut -, sind normalerweise in großem Überschuss vorhanden. Erst bei einer Verminderung um 90% oder mehr einer dieser Komponenten macht sich eine erhöhte Blutungsneigung bemerkbar. Zu lebensbedrohlichen Blutungen kommt es erst, wenn Mangelzustände auftreten, bei denen ein Gerinnungsfaktor bzw. die Blutplättchen nur mehr einige Prozent ihres Normalwertes aufweisen. Die zentrale Bedeutung des Tissue Faktors für die Auslösung des Gerinnungsvorganges und seine weite Verbreitung in allen Organen steht zwar außer Frage, aber in Geweben mit niederem Tissue-Faktorgehalt treten schwere Störungen der Blutgerinnung gerade dann auf, wenn ein Faktor des *intrinsic* Tenasekomplexes oder des Prothrombinasekomplexes pathologisch vermindert ist. Die wichtigsten Beispiele hierfür sind Patienten, die an einer Hämophilie A oder Hämophilie B leiden. Blut dieser Patienten gerinnt in den meisten Fällen noch, aber es kommt wegen der ungenügenden oder fehlenden Ausbildung des *intrinsic* Tenasekomplexes zu einer ungenügenden Thrombinbildung im geronnenen Blut und zur raschen Auflösung desselben, so dass keine ausreichende Hämostase zustande kommt.

Thrombin, meist bovinen Ursprungs, wird als nicht parenteral zu verabreichendes Arzneimittel zur Blutstillung oberflächlicher Verletzungen eingesetzt. Seine hämostyptische Wirkung konnte durch die gemeinsame Anwendung mit fibrinogenhältigen Arzneimitteln entscheidend verbessert werden (Grey; Young et al.). Fibrinogen und Thrombin werden entsprechend ihrer speziesspezifischen Anwendung nunmehr überwiegend aus allogenem Ausgangsmaterial gewonnen. Durch die kombinierte Anwendung von Thrombin mit fibrinogenhältigen Arzneimitteln wird versucht, die physiologische Blutgerinnung und die dabei auftretende Hämostase nachzuvollziehen, bzw. zu verbessern. Dies kann auch bei Patienten mit schweren Blutgerinnungsstörungen erreicht werden (Matras et al.).

Durch die gemeinsame Anwendung von Fibrinogenkonzentraten, deren Fibrinogengehalt das 10- bis 20fache des Blutes beträgt, und hohen Mengen Thrombins (100 - 1000 E pro mL) gelingt es, die Gerinnungszeit in einem solchen Fibrinogen-Thrombingemisch in den Sekundenbereich zu bringen und im Vergleich zur physiologischen Blutungszeit eine 10 bis 100fache Verkürzung zu erlangen. Dadurch wurde es möglich, bei optimaler Applikation solcher Fibrinogen-Thrombingemische praktisch eine sofortige Blutstillung zu erreichen, soweit keine großen Blutgefäße, insbesondere arterielle, verletzt wurden (Spängler).

Das durch Thrombin zu Fibrin umgewandelte Fibrinogen haftet ebenso wie geronnenes Blut am Wundbett an, wobei es offenbar durch die unter Thrombinwirkung aktivierten Transglutaminasen auch zu kovalenten Bindungen zwischen dem verletzten Gewebe und dem gebildeten Fibrin kommt. Diese starke Anhaftung des gebildeten Fibrins am Gewebe kann auch zum Kleben von nicht blutenden Geweben genützt werden, da das gebildete Fibrin in den meisten Fällen die Heilung des geklebten Gewebes nicht stört und innerhalb von Tagen bis Wochen weitgehend abgebaut wird (Matras et al.).

Im Gegensatz zur möglichst raschen Einleitung des Gerinnungsvorganges bei der Blutstillung mit Fibrinogen-Thrombingemischen ist bei der Klebung von Gewebeteilen und bei Abdichtungen ein verzögerter Beginn des Gerinnungsvorganges erwünscht. Dadurch können die zu klebenden Gewebsteile besser adaptiert werden, und ähnliche Adaptierungen sind auch bei Abdichtungen erforderlich. Bisher wurde die Verzögerung des Gerinnungsvorganges durch Verminderung der Thrombinkonzentration auf etwa 1% der Thrombinmenge, die zur Blutstillung verwendet wird, bewerkstelligt. Jedoch bringen sowohl die Anwendung hoher als auch niederer Thrombinkonzentrationen verschiedene Nachteile mit sich.

Hochviskose Fibrinogenlösungen mit einem Fibrinogengehalt zwischen 5 und 10% können mit Thrombinmengen zwischen 100 und 1.000 Einheiten innerhalb von Sekunden zur Gerinnung gebracht werden. Diese kurze Gerinnungszeit ist erforderlich, damit nach Aufbringung eines solchen Gemisches auf eine blutende Stelle die Gerinnung rasch einsetzt und die Blutung dadurch zum Stillstand gebracht wird. Der Nachteil einer solchen Vorgangsweise ist die schlechte Durchmischung der Fibrinogenlösung mit der Thrombinlösung, da die hohe Viskosität des Gemisches eine komplette Durchmischung in kurzer Zeit nicht erlaubt. Dies bewirkt eine inhomogene Gerinnung und damit eine Benachteiligung der biomechanischen Qualität.

Andererseits besteht bei der Anwendung von Fibrinogen-Thrombingemischen, die nicht der Blutstillung, sondern der Klebung von Gewebeteilen dienen, meistens die Notwendigkeit, das Auftreten der Gerinnung im Fibrinogen-Thrombingemisch zu verzögern, um die zu klebenden Gewebeteile oder Abdichtungen optimal adaptieren zu können, bevor die Gerinnung eintritt. Dies wird derzeit durch Reduktion der Thrombinmenge auf ein Zehntel bis auf ein Hundertstel der für die Hämostase verwendeten Quantität Thrombin versucht, allerdings mit dem Nachteil, dass nicht alles vorhandene Fibrinogen in Fibrin und Faktor XIII in XIIIa übergeführt werden kann. Auch bei dieser Vorgangsweise kann keine optimale Ausbildung eines Fibringerinnsels erhalten werden, da die niedere Thrombinkonzentration nicht ausreicht, um TAFI in TAFla überzuführen.

Eine weitere Problematik stellt die Verwendung von bovinem Material zur Herstellung von thrombinhältigen Arzneimitteln dar. Da die mögliche Übertragung von Prionen durch jegliche, von Rindern verwendete Organe nicht mit Sicherheit ausgeschlossen werden kann, ist heute die Anwendung von bovinem Thrombin stark zurück gegangen.

Bovines Thrombin hat den weiteren Nachteil, dass es für andere Spezies antigen wirkt und Allergien und Anaphylaxien verursachen kann. Des Weiteren wurden auch Blutgerinnungsstörungen bei mit bovinem Thrombin behandelten Patienten beobachtet, da Thrombin die Bildung von Antikörpern gegen Gerinnungsfaktoren verursachen kann, die mit menschlichen Gerinnungsfaktoren kreuzreagieren und so die Blutgerinnung verzögern können.

Bei der Herstellung von Thrombin aus Prothrombin wurde wegen der guten Ausbeute häufig zur Aktivierung des Thrombins Thromboplastin, das aus tierischem Ausgangsmaterial, meist Rinderhirn, hergestellt war, verwendet. Wegen der Möglichkeit der Übertragung von Prionen wird Thromboplastin aus tierischem Ausgangsmaterial weitgehend zur Thrombinherstellung ausgeschlossen und eine schlechtere Ausbeute von Thrombin in Kauf genommen.

Die Erfindung stellt sich daher u.a. die Aufgabe, eine virussichere, thrombingenerierfähige, pharmazeutischen Wirkstoffzubereitung zur Verfügung zu stellen, die
1. zu einem thrombingenerierfähigen Arzneimittel formuliert und mit einem fibrinogenhältigen Arzneimittel vermischt, eine komplette und homogene Mischung dieser Komponenten ergibt, ohne dass eine komplette Vermischung durch zu schnelle Gerinnung verhindert wird. Es soll aber nicht nur die vollständige Durchmischung, sondern auch die Adaptierung der zu versiegelnden oder abzudichtenden Gewebsteile ermöglicht werden, bevor die Gerinnung einsetzt. Die Thrombinbildung muss sich auch nach der einsetzenden Gerinnung weiter fortsetzen, um alles im Gerinnsel vorhandene Fibrinogen, Faktor XIII und TAFI in Fibrin, Faktor XIIIa und TAFIa überzuführen, um einen dauerhaften, durch Fibrin gebildeten Wundverschluss zu gewährleisten, und in der
2. das enthaltene Prothrombin möglichst vollständig in Thrombin übergeführt und dieses nach weiterer Reinigung zu einem thrombinhältigen Arzneimittel verarbeitet werden kann. Dieses Arzneimittel kann als solches oder gemeinsam mit anderen, fibrinogenhältigen Arzneimitteln verwendet werden.

Die aus den thrombingenerierfähigen, pharmazeutischen Wirkstoffzubereitungen hergestellten Arzneimittel sollen je nach Anwendung bei einer bestimmten Spezies nur allogenes Material dieser Spezies enthalten und ebenso sollen bei der Herstellung nur speziesspezifische Proteine verwendet werden. Alle verwendeten Ausgangsmaterialien und Hilfsmaterialien sollen die Übertragungsmöglichkeit von Prionen ausschließen. Die Thrombingenerierung aus Prothrombin *in vivo* und *in vitro* soll so vollständig wie möglich erfolgen, da nur das im Gerinnsel vorhandene Prothrombin zur Bildung von weiterem Thrombin in dem bereits geronnenen Fibrinogen zur Verfügung steht.

Die erfindungsgemäße pharmazeutische Wirkstoffzubereitung dient zur Herstellung eines thrombingenerierfähigen oder thrombinhältigen Arzneimittels und ist dadurch gekennzeichnet, dass sie enthält:
(A) aus Plasma oder gentechnologisch gewonnenes Prothrombin (Gerinnungsfaktor II),
(B) aus Plasma oder gentechnologisch gewonnene Gerinnungsfaktoren V, VIII, IX, X, die zumindest teilweise in aktiviertem Zustand vorliegen können, und aus Plasma oder gentechnologisch gewonnenen Gerinnungsfaktor Xla, und
(C) prionensichere, gerinnungsaktive Phospholipide, welche gegebenenfalls in Liposomen enthalten sind.

Die erfindungsgemäße Wirkstoffzubereitung enthält bevorzugt aus Plasma oder gentechnologisch gewonnenen Gerinnungsfaktor VII oder ein Gemisch von aus Plasma oder gentechnologisch gewonnenen Gerinnungsfaktoren VII und Vlla. Ferner enthält sie bevorzugt einen gentechnologisch hergestellten Tissue Faktor als solchen oder in relipidierter Form.

Die erfindungsgemäße Wirkstoffzubereitungkann auch aus zwei oder mehreren Plasmafraktionen und den gerinnungsaktiven Phospholipiden bestehen, wobei die einzelne Plasmafraktion einen oder mehrere Gerinnungsfaktoren bzw. aktivierte Gerinnungsfaktoren der Komponenten (A) und (B) enthält. Das gesamte Plasmafraktionengemisch muß jedoch alle, in den Komponenten (A) und (B) enthaltenen Gerinnungsfaktoren bzw. aktivierte Gerinnungsfaktoren enthalten, wobei vorzugsweise noch Tissue-Faktor zugesetzt wird.

Die Gerinnungsfaktoren sind insbesondere ausschließlich aus Blutplasma einer bestimmten Säugetierspezies oder aus den entsprechenden gentechnologisch gewonnenen Gerinnungsfaktoren oder aktivierten Gerinnungsfaktoren, einschließlich Tissue Faktor, hergestellt.

In der erfindungsgemäßen pharmazeutische Wirkstoffzubereitung sind vorteilhaft das Prothrombin, die Gerinnungsfaktoren V, VIII, IX, X, XIa und gegebenenfalls die Gerinnungsfaktoren VII und Vlla, sowie der Tissue Faktor durch Virusinaktivierung und/oder Virusabreicherung virussicher.

Die erfindungsgemäße pharmazeutische Wirkstoffzubereitung kann in tiefgefrorener oder gefriergetrockneter Form vorliegen.

Die Erfindung betrifft ferner ein thrombingenerierfähiges Arzneimittel, welches aus einer erfindungsgemäßen pharmazeutischen Wirkstoffzubereitung herstellbar ist.

Das erfindungsgemäße thrombingenerierfähige Arzneimittel kann in tiefgefrorenem oder in gefriergetrocknetem Zustand vorliegen.

Eine bevorzugte Ausführungsform des erfindungsgemäßen thrombingenerierfähigen Arzneimittels ist dadurch gekennzeichnet, dass es zusätzlich Thrombin in einer solchen Menge enthält, dass es nach Auftauen oder Rekonstitution nicht mehr als 1 Einheit Thrombin pro ml enthält.

Die Erfindung betrifft ferner ein thrombinhältiges Arzneimittel, das aus einer erfindungsgemäßen thrombingenerierfähigen, pharmazeutischen Wirkstoffzubereitung herstellbar ist. Es kann in tiefgefrorenem oder gefriergetrockneten Zustand vorliegen.

Die Erfindung betrifft auch ein Arzneimittelgemisch, das vor seiner Anwendung durch Vermischen eines erfindungsgemäßen Arzneimittels mit einem fibrinogenhältigen Arzneimittel innerhalb von 30 bis 300 Sekunden gerinnt.

Die Erfindung betrifft darüberhinaus auch ein thrombinhältiges Arzneimittel, das mit dem obigen Arzneimittelgemisch versetzt, innerhalb von 3 bis 10 Sekunden gerinnt.

Eine bevorzugtes Verfahren zur Herstellung einer erfindungsgemäßen thrombinhältigen pharmazeutischen Wirkstoffzubereitung ist dadurch gekennzeichnet, dass eine erfindungsgemäße thrombingenerierfähige Wirkstoffzubereitung mit Ca-lonen versetzt wird, wobei ein thrombinhältiges Produkt gebildet wird, welches anschließend chromatographisch gereinigt wird.

Eine vorteilhafte Ausführungsform des erfindungsgemäßen Verfahrens besteht darin, dass die Wirkstoffzubereitung durch Virusinaktivierung während oder nach der Thrombinbildung, sowie durch Virusabreicherung nach abgeschlossener Thrombinbildung virussicher gemacht wird.

Die Erfindung betrifft auch ein Verfahren zur Herstellung eines Pro-Kofaktorenkonzentrates enthaltend die Gerinnungsfaktoren V und VIII, und ist dadurch gekennzeichnet, dass aus Plasma gewonnenes Kryopräzipitat gelöst und aus der Lösung Fibrinogen abgetrennt wird, wobei eine die Gerinnungsfaktoren V und VIII hältige Lösung erhalten wird, welche mit Calciumchlorid versetzt werden kann.

Ein weiteres erfindungsgemäßes Verfahren betrifft die Herstellung eines Gerinnungsfaktor-XI-hältigen Prothrombinkomplexkonzentrates enthaltend die Gerinnungsfaktoren II, VII, Vlla, IX, X und XI, wobei pro 1,0 µg Prothrombin mindestens 0,030 µg Gerinnungsfaktor Xla enthalten sind, und ist dadurch gekennzeichnet, dass aus Plasma gewonnener Kryopräzipitatüberstand an einem Anionenaustauscher adsorbiert und nach Waschen des Adsorbats und Lagerung in der Kälte Gerinnungsfaktor-XIa-hältiger Prothrombinkomplex eluiert wird.

Die Erfindung beruht auf der Erkenntnis, dass eine praktisch komplette, zeitlich steuerbare Bildung von α-Thrombin aus virussicherem Prothrombin durch einen virussicheren *intrinsic* Tenase-Prothrombinasekomplex erfolgt. Der *intrinsic* Tenase-Prothrombinasekomplex wird durch Aktivierung der entsprechenden virussicheren, bzw. virussicher gemachten Gerinnungsfaktoren in Gegenwart optimaler Calonenkonzentrationen mit prionensicheren, gerinnungsaktiven Phospholipiden und der virussicheren Aktivatorsubstanz Gerinnungsfaktor Xla und/oder die virussicheren Aktivatorsubstanzen Gerinnungsfaktor VIIa und Tissue-Faktor gebildet.

Die Aufgabenstellung wird somit erfindungsgemäß mit einer aus drei Komponenten (Komponenten A, B und C) bestehenden, pharmazeutischen Wirkstoffzubereitung zur Herstellung eines virussicheren, thrombingenerierfähigen oder thrombinhältigen Arzneimittels gelöst, wobei die pharmazeutische Wirkstoffzubereitung dadurch gekennzeichnet ist, dass
die Komponente A ein aus Plasma oder gentechnologisch hergestelltes, virussicheres Prothrombin als pharmazeutisch aktive Substanz,
die Komponente B virussichere, aus Plasma oder gentechnologisch gewonnene Gerinnungsfaktoren V, VIII, IX, X, XIa als pharmazeutisch aktive Substanzen und, falls zusätzlich zur Bildung der Prothrombinase- und *intrinsic* Tenasekomplexe auch die Bildung von *extrinsic* Tenasekomplex veranlasst werden soll, noch die virussicheren Gerinnungsfaktoren VII und/oder Vlla sowie Tissue Faktor als pharmazeutisch aktive Substanzen, und
die Komponente C vollsynthetisch hergestellte, gerinnungsaktive Phospholipide als pharmazeutisch aktive Substanzen mit einem über 15° C liegenden Umwandlungspunkt der parakristallinen in die flüssig kristalline Form enthält. Ein Gemisch der in den Komponenten (A) und (B) enthaltenen, pharmazeutisch aktiven Substanzen, d.h. die Gerinnungsfakoren bzw. aktivierten Gerinnungsfaktoren, kann auch durch Mischen zweier oder mehrerer Plasmafraktionen erhalten werden, in denen alle erforderlichen, pharmazeutisch aktiven Substanzen enthalten sind und denen Tissue-Faktor erforderlichenfalls zugesetzt werden kann.

Nach Mischung der drei Komponenten und Zugabe einer optimalen CaCl₂-Menge wird temperaturabhängig zwischen 0 ° und 40 °C Thrombin gebildet, wobei die Geschwindigkeit der Thrombinbildung mit steigender Temperatur zunimmt.

Die Herstellung der Komponente A erfolgt durch Auflösen von gefriergetrocknetem, virussicherem Prothrombin oder Auftauen einer eingefrorenen Lösung von virussicherem Prothrombin und der Zubereitung einer Prothrombinstammlösung mit 10 - 100 Einheiten Prothrombin pro mL und einem Gehalt von 0,1% Natriumzitrat bei pH 7,3.

Die Wertbemessung der Stammlösung erfolgt durch Bestimmung der Prothrombineinheiten mit Hilfe eines Gerinnungsfaktor-II-Mangelplasmas, mit dem eine Eichkurve unter Verwendung eines Referenz-Normalplasmas erstellt wurde.

Mit Hilfe von Ecarin wird die maximale Menge Meizothrombin/Thrombin bestimmt, die von einer Einheit Prothrombin generierbar ist. Die Meizothrombin/ Thrombinbestimmung erfolgt mit dem chromogenen Substrat S-2238. Weiters wird die Menge Thrombin in NIH-Einheiten bestimmt, die aus einer Einheit Prothrombin nach Thromboplastinzusatz generiert wird. Eine entsprechende Standardkurve zur Thrombinbestimmung wird mit Hilfe des internationalen α-Thrombinstandards errichtet.

Die Komponente B wird durch Vermischen von Stammlösungen virussicherer Gerinnungsfaktoren V, VIII, IX, X, Xla, sowie der Gerinnungsfaktoren VII, VIIa und Tissue Faktor hergestellt. Die Konzentrationen der einzelnen Gerinnungsfaktoren in ihren Stammlösungen liegen zwischen 10 - 100 Einheiten pro mL; die Tissue-Faktor-Stammlösung enthält 25 µg/mL. Anstelle einer Stammlösung von Gerinnungsfaktor V kann auch eine Stammlösung von Gerinnungsfaktor Va verwendet werden. Der Gehalt eines einzelnen Gerinnungsfaktors in einer Stammlösung wird mit Hilfe der entsprechenden Mangelplasmen bestimmt und zur Bestimmung jedes einzelnen Gerinnungsfaktors eine Standardkurve mit gepooltem Normalplasma errichtet.

Um festzustellen, welche Mengen der einzelnen Gerinnungsfaktoren erforderlich sind, um noch eine optimale Thrombingenerierung zu erhalten, werden Mischungen der Komponenten A und B hergestellt, die je eine Einheit der Gerinnungsfaktoren II, V, VIII, IX, X, Xla und II, sowie Xla, VII, VIIa mit 2,5 µg Tissue Faktor pro mL des Ansatzes enthalten. Nach Zusatz der Liposomenemulsion und nachfolgender Rekalzifizierung wird die Thrombinbildung bei 26°. C in entsprechenden Zeitintervallen bestimmt. Ebenso wird in diesen Ansätzen der Prothrombinverbrauch und die Bildung von β- und γ-Thrombin festgestellt. Die Überführung von Prothrombin in Thrombin wird in Anwesenheit von 0,1% PEG vorgenommen und jene Mengen Gerinnungsfaktoren und Tissue Faktor pro mL bestimmt, die erforderlich sind, um noch eine optimale Thrombingenerierung zu erreichen.

Gemische von Komponenten A und B können auch aus zwei oder mehreren Plasmafraktionen erhalten werden, soweit sie alle Gerinnungsfaktoren und aktivierten Gerinnungsfaktoren, die zur Bildung der *intrinsic* Tenase- und Prothrombinasekomplexe erforderlich sind, als pharmazeutisch aktive Substanzen enthalten. Die Aktivierung des *intrinsic* Tenase-Pathways erfolgt entweder durch den Gerinnungsfaktor Xla oder über den *extrinsic* Tenase-Pathway, insbesondere wenn kein TFPI vorhanden ist. Es ist auch möglich, den *intrinsic* Tenase-Pathway gleichzeitig durch den Gerinnungsfaktor XIa und über den *extrinsic* Tenase-Pathway zu aktivieren. In einem Gemisch von Gerinnungsfaktoren, die zur Bildung des *intrinsic* Tenasekomplexes erforderlich sind, kann sowohl durch Zusatz, bzw. Vorhandensein des Gerinnungsfaktors Xla bestimmt werden, ob mindestens 90% des vorhandenen Prothrombins in Thrombin übergeführt werden, und wenn dies nicht der Fall ist, welche Mengen Faktor Vlla und Tissue Faktor erforderlich sind, um eine solche praktisch vollständige Umsetzung von Prothrombin in Thrombin zu erreichen. Zur Thrombingenerierung wird ein Überschuss von gerinnungsaktiven Phospholipiden verwendet. Die Aktivierung wird entweder bei 26° C oder 37° C und einer optimalen Ca-Ionenkonzentration durchgeführt.

Die Virussicherheit der verwendeten Gerinnungsfaktoren, bzw. der gerinnungsfaktorhältigen Plasmafraktionen, wie auch des gentechnologisch gewonnenen Tissue Faktors wird durch Virusinaktivierung, z.B. nach dem Solvent/Detergent-Verfahren, und nachfolgende Virusabreicherung mittels Nanofiltration erreicht.

Die Komponente C wird aus Mischungen von vollsynthetischen, und dadurch prionensicheren, Cholin- oder Serinphospholipiden oder aus Cholin-Serin-Äthanolaminphospholipiden hergestellt. Es werden nur Phospholipide verwendet, deren Phasenübergangstemperatur vom parakristallinen Gelzustand in den flüssigkristallinen Zustand zwischen 15° und 40° C liegt. Aus den Phospholipidgemischen können Emulsionen hergestellt werden, die Liposome mit einem Durchmesser von 20 nm bis 1.000 nm aufweisen und die polaren Anteile der Phospholipide in einer äußeren Membran ausgebildet haben. Mit Hilfe von Temperaturen zwischen 50° C und 100° C können Liposome mit einem Durchmesser von 20 nm - 200 nm hergestellt werden. Solche Emulsionen können mit Hilfe bakteriendichter Filter sterilisiert, steril abgefüllt, getrocknet und unter Stickstoff aufbewahrt und gelagert werden. Durch Zusatz von Wasser können die gefriergetrockneten Emulsionen rekonstituiert werden.

Die Virussicherheit der verwendeten Gerinnungsfaktoren und des nicht relipidierten Tissue Faktors wird durch Virusinaktivierung, vorzugsweise mit dem Solvent/ Detergent-Verfahren und nachfolgender, weitgehender Entfernung des Solvent/Detergent erreicht. Quantitierbare Spuren des verwendeten Solvents, Trinitrobutylphosphat, und des Detergents, Tween 80 verbleiben in der thrombinhältigen Lösung. Die Virusabreicherung erfolgt vorzugsweise durch Nanofiltration nach vorheriger Klärfiltration, die stufenweise mit immer enger porigen Filtern erreicht wird, beginnend mit einer Porenweite von 5.000 nm, absteigend bis 35 nm. Vorzugsweise kann nach der Filtration durch einen 35 nm Nanofilter noch durch Nanofilter mit Porenweiten von 20 nm und 15 nm filtriert werden.

Die Thrombingenerierung dieser pharmazeutischen Wirkstoffzubereitung ist stark temperaturabhängig. Daher ist es möglich, durch Vermischen der Komponenten A, B, und C, selbst bei optimaler Ca-Ionenkonzentration, die Thrombinbildung aus Prothrombin hintanzuhalten, sofern bei niederen Temperaturen um den Gefrierpunkt gearbeitet wird. Zur Lagerung kann die erfindungsgemäße Wirkstoffzubereitung tiefgefroren werden. Sie kann aber auch gefriergetrocknet und bei Temperaturen zwischen 0° C und 25° C gelagert werden.

Die thrombingenerierfähige, pharmazeutische Wirkstoffzubereitung kann bei niederen Temperaturen zu einem Arzneimittel mit einer Thrombingenerierungskapazität von vorzugsweise 100 bis 1.000 Einheiten Thrombin pro mL formuliert, steril filtriert und portioniert abgefüllt werden.

Die thrombingenerierfähige, pharmazeutische Wirkstoffzubereitung wird auch zur Herstellung von thrombinhältigen Arzneimitteln verwendet. Die Thrombingenerierung wird vorzugsweise bei Temperaturen zwischen 20° C bis 40° C durchgeführt.

Für die Herstellung von Thrombin aus einer thrombingenerierfähigen, pharmazeutischen Wirkstoffzubereitung, bei der noch keiner oder nicht alle Komponenten einer Virusinaktivierung unterzogen waren, kann die Virusinaktivierung während oder nach der Thrombingenerierung durchgeführt werden. Die Entfernung der virusinaktivierenden Substanzen kann gemeinsam mit einer anschließenden Reinigung des generierten Thrombins erfolgen. Es ist möglich, eine thrombinhältige, pharmazeutische Wirkstoffzubereitung mit einem Thrombingehalt von 1.000 bis 10.000 Einheiten pro mL zu erhalten, die durch Klärfiltrationen und nachfolgende Nanofiltration virusabgereichert werden kann. Die virussichere Wirkstoffzubereitung kann dann formuliert und sterilfiltriert zu einem Arzneimittel verarbeitet werden.

Die Verwendung von thrombingenerierfähigen und/oder thrombinhältigen Arzneimitteln gemeinsam mit fibrinogenhältigen Arzneimitteln kann zur Hämostase, zur Gewebeklebung in Kombination mit Nähten oder zur Abdichtung von Hohlräumen und Gefäßen gegen Austritt von Gasen und Flüssigkeiten verwendet werden.

Durch Vermischen eines thrombingenerierfähigen Arzneimittels mit einem fibrinogenhältigen Arzneimittel und eventuellen Zusatz geringer Mengen Thrombin kann die Gerinnungszeit eines solchen Gemisches auf 30 bis 300 Sekunden eingestellt werden. Dadurch ist eine komplette Vermischung der Arzneimittel selbst bei hoher Viskosität einer solchen Mischung möglich. Ebenso steht nach vollständiger Durchmischung der Arzneimittel ein genügend langer Zeitraum für die erforderliche Adaptierung der Gewebeteile, die geklebt oder abgedichtet werden sollen, zur Verfügung. Nach Eintreten der Gerinnung kommt es dann - ähnlich wie bei dem physiologischen Gerinnungsvorgang - zu einer weiteren Bildung von Thrombin im bereits teilweise geronnenen Fibrinogen, so dass nach einiger Zeit - bis zu mehreren Stunden - praktisch alles Prothrombin in Thrombin umgewandelt ist und dieses das gesamte Fibrinogen in Fibrin überführt. Eine solche fortgesetzte Thrombinbildung ermöglicht nicht nur die homogene Ausbildung von Fibrin im bereits geronnenen Fibrinogen, sondern auch die gleichförmige Aktivierung von Faktor XIII und die dadurch einsetzende Quervernetzung des gebildeten Fibrins, ebenso wie die Aktivierung von TAFI und die dadurch erfolgende Abspaltung der endständigen Lysinreste des Fibrins.

Die Anwendung fibrinogenhältiger Gemische zur Hämostase erfordert eine sehr kurze Gerinnungszeit. Um diese zu erreichen und trotzdem eine homogene Ausbildung des geronnenen Fibrinogens und die Aktivierung von Zymogenen, wie Faktor XIII und/oder TAFI, zu gewährleisten, können fibrinogenhältige Arzneimittel komplett mit thrombingenerierfähigen Arzneimitteln vermischt werden und solche Gemische dann zusammen mit einer Thrombinlösung, die einen hohen Gehalt an Thrombin aufweist, auf die blutende Stelle aufgebracht werden. Obwohl die einsetzende Gerinnung zunächst nicht zu einem homogenen Fibrinklot führt, setzt die nachfolgende Thrombingenerierung des im Fibrinklot enthaltenen thrombingenerierfähigen Arzneimittels die Überführung von Prothrombin in Thrombin fort, so dass es schließlich zu einem homogen geronnenen Fibrinklot mit homogen verteiltem Faktor Xllla und homogen verteiltem TAFIa kommt. Dadurch ist es möglich, auch bei schnell gerinnenden Mischungen von thrombinhältigen mit fibrinogenhältigen Arzneimitteln eine ausgezeichnete Fibrinstruktur und eine besondere Resistenz gegenüber fibrinolytischen Einflüssen zu erzielen.

Eine weitere Möglichkeit, in einem thrombingenerierfähigen Gerinnungsfaktorengemisch, bestehend aus Prothrombinkomplex und Faktor VIII, eine spontane Thrombinbildung auszulösen, ist die Zugabe von Faktoren des Kontaktsystems.

Hierzu müssen die Gerinnungsfaktoren XI und XII, soweit sie nicht bereits in dem Gemisch vorhanden sind, zugesetzt werden.

Die Aktivierung des Gerinnungsfaktors XI *in vivo* ist noch nicht in allen Einzelheiten vollständig geklärt. Fest steht, dass Blutzellen und Zellen des Endothels bei diesem Aktivierungsvorgang eine wesentliche Rolle spielen. Faktor XI ist im Blut in nicht kovalenter Form an hochmolekulares Kininogen (K_{d}10⁻⁸) und Prothrombin (K_{d}10⁻⁷) gebunden. Diese Komplexe können sich an aktivierte Plättchen anlagern, wobei es in Anwesenheit von Thrombin und/oder Gerinnungsfaktor Xlla zu einer Aktivierung des Gerinnungsfaktors XI kommt.

*In vitro* kann die Aktivierung des Gerinnungsfaktors XI durch Gerinnungsfaktor Xlla an negativ geladenen Oberflächen erfolgen, indem Gerinnungsfaktor XIIa durch Kallikrein aus Gerinnungsfaktor XII gebildet wird. Die Bildung von Kallikrein aus dem im Blut vorhandenen Präkallikrein kann durch unterschiedliche Mechanismen erfolgen, wie z.B. durch Präkallikreinaktivator oder Metallsalze der Ellagsäure.

Erfindungsgemäß kann in einem thrombinbildungsfähigen Gerinnungsfaktorengemisch, bestehend aus Prothrombinkomplex und Gerinnungsfaktor VIII, eine spontane Thrombingenerierung durch Aktivierung des darin enthaltenen Gerinnungsfaktors XI ausgelöst werden, wobei alle Komponenten dieses Gemisches vorher virusinaktiviert wurden. Vorzugsweise wird diese Aktivierung in Anwesenheit von prionensicheren, gerinnungsaktiven Phospholipiden mit virussicherem Kallikrein durchgeführt. Anstelle von Kallikrein kann auch virusinaktiviertes Präkallikrein mit virusinaktiviertem Präkallikreinaktivator oder -aktivatoren, wie z.B. Metallsalzen der Ellagsäure, verwendet werden.

Für die Herstellung thrombinhältiger, pharmazeutischer Wirkstoffe kann die Virusinaktivierung auch während oder nach der Thrombingenerierung erfolgen.

Mit den nachfolgenden Beispielen wird die Erfindung näher beschrieben.

### Beispiele

1. Herstellung der Komponente A.
   100 mg virussicheres Prothrombin wird in 1 L 0,1%iger Natriumzitratlösung bei pH 7,5 gelöst und der Gehalt an Prothrombin pro mL mit Hilfe eines Prothrombinmangelplasmas bestimmt. Ebenso wird nach Ecarinzusatz die gebildete Menge Meizothrombin und Thrombin durch Umsetzung von chromogenem Substrat S-2238 bestimmt. Weiters wird dieThrombinbildung mit Hilfe einer Prothrombinasepräparation durchgeführt. Das gebildete Thrombin wird mit Hilfe einer Fibrinogenlösung als Thrombinzeit bestimmt. Mit dieser Fibrinogenlösung wurde vorher eine Standardkurve mit Hilfe des internationalen Standards für α-Thrombin errichtet. Diese Prothrombinstammlösung kann eingefroren bei -20° C minidestens sechs Monate gelagert werden.
2. Herstellung der Komponente B aus einzelnen Gerinnungsfaktoren.
   Virussichere, hochgereinigte Gerinnungsfaktorenkonzentrate, die aus Plasma oder gentechnologisch gewonnen wurden, werden in 0,1%igem Natriumzitrat pH 7,5 gelöst und unter Rühren vermischt: 3,2 mg Faktor V, 0,2 mg Faktor VIII, 5,0 mg Faktor IX, 11 mg Faktor X und 0,5 mg Faktor Xla. Nach Probenziehung wird die Lösung eingefroren und bei -20° C oder darunter liegenden Temperaturen gelagert. An einer äquivalenten Probe wird die Menge einer 1 M CaCl₂-Lösung bestimmt, die erforderlich ist, um die Ca-Ionenaktivität äquivalent zu einer 5 mM - 8 mM CaCl₂-Lösung zu bringen.
3. Herstellung eines Gemisches der Komponenten A und B.
   Die eingefrorenen Lösungen der Komponente A, hergestellt nach Beispiel 1, und der Komponente B, hergestellt nach Beispiel 2, werden vorsichtig aufgetaut, wobei die Temperatur nicht über 4° C ansteigen soll. Aus den erhaltenen Lösungen wird eine Mischung aus gleichen Teilen der Komponente A und der Komponete B hergestellt, die erforderlichen Proben werden gezogen, und das Gemisch der Komponenten wird wieder eingefroren und bei -20° C gelagert.
4. Herstellung eines Gemisches aus Plasmafraktionen, das die Gerinnungsfaktoren der Komponenten A und B enthält.
   Aus 10 L tiefgefrorenem Source Plasma wird nach vorsichtigem Auftauen; wobei eine Temperatur von 4° C nicht überschritten wird, durch Zentrifugation bei 6.000 g über 15 Minuten das Kryopräzipitat vom Kryopräzipitatüberstand abgetrennt.
   a. Gerinnungsfaktor-Xla-hältiges Prothrombinkomplexkonzentrat:
      Der Kryopräzipitatüberstand wird mit 16 g schwachem Anionenaustauscher DEAE-Sephadex A-50 versetzt, der pH Wert zwischen 7,8 und 8,8 mit 0,1 normaler Na-Lauge eingestellt und eine Stunde bei einer Temperatur zwischen 3° C bis 6° C gerührt. Das Gemisch kann längstens 24 Stunden bei dieser Temperatur gehalten werden, bevor der Ionenaustauscher durch Filtration und/oder Zentrifugation abgetrennt wird. Die vom Ionenaustauscher befreite Flüssigkeit wird zur Gewinnung weiterer Plasmafraktionen aufbewahrt und der abgetrennte Ionenaustauscher zur Gewinnung des Prothrombinkomplexes weiterverarbeitet. Die Lagerung des so abgetrennten Ionenaustauschers kann bis zu 100 Stunden bei einer Temperatur zwischen 4° und 6° C erfolgen. Zur Weiterverarbeitung wird der abgetrennte Ionenaustauscher zwei Mal mit je 1 L 0,5%iger Kochsalzlösung gewaschen und der Gerinnungsfaktor-Xla-hältige Prothrombinkomplex durch Elution des Ionenaustauschers mit 1.000 mL einer 0,3 M NaCI-Lösung bei pH 7,5 gewonnen. Der Ionenaustauscher wird nach der Elution nochmals mit 500 mL 0,3 M Kochsalzlösung bei pH 7,5 gewaschen und die Waschflüssigkeit mit dem Eluat vereinigt. In diesem Gesamteluat wird mit Hilfe von Ecarin die vorhandene Menge Prothrombin bestimmt und das Gesamteluat durch Verdünnung auf den gewünschten Prothrombingehalt, der zwischen 2 und 5 Prothrombineinheiten pro mL liegen kann, gebracht. Das Gesamteluat als Gerinnungsfaktor-XIa-hältiges Prothrombinkomplexkonzentrat kann nach dem Einfrieren in gefrorenem Zustand bei -20° C gelagert werden. Mit Hilfe von Proben, die vor dem Einfrieren entnommen wurden, wird der Gehalt an Gerinnungsfaktoren und aktivierten Gerinnungsfaktoren bestimmt.
   b. Herstellung eines Pro-Kofaktorenkonzentrates:
      Das aus Plasma durch Zentrifugation sedimentierte Kryopräzipitat wird in 750 mL 0,3%igem Zitratpuffer pH 7,0 gelöst und mit 110 g Glyzin versetzt. Nach einstündigem Rühren bei einer Temperatur zwischen 0° C und 2° C wird das ausgefällte Fibrinogen durch Zentrifugation bei 3.000 g mit einer Dauer von 15 Minuten abgetrennt und der Überstand, der die Gerinnungsfaktoren V und VIII enthält, eingefroren. In einer mit Wasser 1:10 verdünnten Probe des Überstandes wird der Gehalt an Faktor VIII bestimmt, ebenso die Menge CaCl₂, die zur Rekalzifizierung erforderlich ist, um eine Ionenaktivität äquivalent zu einer 5mM CaCL₂-Lösung zu erhalten. Die eingefrorene Lösung kann bei -20° C gelagert werden.
   c. Herstellung eines Gemisches aus Gerinnungsfaktor-Xla-hältigem Prothrombinkonzentrat und Pro-Kofaktorenkonzentrat:
      Eine mit destilliertem Wasser 1:10 verdünnte, rekalzifizierte Probe des nach Beispiel 4 b. hergestellten Pro-Kofaktorenkonzentrates wird in Mengen von 0,1; 0,2; 0,4 und 0,8 mL zu je 1 mL dem nach Beispiel 4 a. hergestellten Prothrombinkonzentrat zugesetzt. Diesen Gemischen wird je 0,1 mL einer nach Beispiel 5 hergestellten Liposomenemulsion zugefügt. Nach. Inkubation von 8 Stunden bei 26° C wird jene Menge des Pro-Kofaktorenkonzentrats bestimmt, die erforderlich ist, um 90% oder mehr des vorhandenen Prothrombins in Thrombin überzuführen.
      Zur Herstellung des Gemisches aus Gerinnungsfaktor-Xla-hältigem Prothrombinkomplexkonzentrat und Pro-Kofaktorenkonzentrat, die aus 10 L tiefgefrorenem Plasma gewonnen und eingefroren gelagert sind, werden diese Konzentrate nach dem Auftauen entsprechend in dem im Vorversuch gefundenen, optimalen Verhältnis vermischt. Diese Mischung wird eingefroren und bei -20° C gelagert.
5. Herstellung der Komponente C ohne Emulgator.
   Es werden vollsynthetische und daher virus- und prionensichere Phospholipide verwendet. 200 mg 2Na-1,2-Di-Oleoyl-sn-Glycero-3-Phospho-L-Serin, 400 mg 1,2,Di-Oleoyl-sn-Glycero-3-Phosphocholin und 400 mg Di-Oleoyl-Äthanolamin-Phospholipid werden in 10 mL Chloroform gelöst und die Lösung in einem 250 mL Rundkolben durch Erwärmung und laufendes Drehen des Kolbens zur Trockerne eingedampft. Mit Hilfe eines Stickstoffstroms werden die Reste von Chloroform vollständig entfernt und 10 mL einer 0,1%igen Zitratpufferlösung pH 7,3 zugefügt. Der sich an der Innenwand des Rundkolbens befindliche Phospholipidfilm wird komplett in der Pufferlösung bei 65° C emulgiert und bei der Emulgierung so lange wiederholt mit einer Vortex-Vorrichtung geschüttelt, bis der ganze Lipidfilm von der inneren Oberfläche des Kolbens verschwunden ist. Die Emulsion wird als solche oder 1:10 mit 0,1%igem Zitratpuffer pH 7,0 verdünnt, durch ein Filter mit einem Porendurchmesser von 0,45 µm filtriert und nachfolgend durch ein Filter mit einer Porenweite von 0,22 µm sterilfiltriert. Diese Liposomenemulsion kann steril im Kühlschrank gelagert werden und wird vor Gebrauch mit einer Vortex-Vorrichtung 1 Minute geschüttelt. Die Emulsion wird entsprechend der europäischen Pharmakopöe auf Sterilität und Pyrogenfreiheit geprüft (Eur. Pharm. 4th Edition 2002 Seiten 123-126, 2.6.1. Sterility. Seiten 131 - 132, 2.6.8. Pyrogens).
6. Herstellung der Komponente C mit Natriumcholat.
   250 mg 2Na-1,2-Di-Oleoyl-sn-Glycero-3-Phospho-L-Serin, 750 mg 1,2, Di-Oleoyl-sn-Glycero-3-Phosphocholin und 500 mg Natrium-Cholat werden in 10 mL einer 1 + 1 Mischung von Chloroform und Methylalkohol gelöst und beide Lösungsmittel in Vakuum abgedampft, in einer Weise, dass ein dünner, gleichmäßiger Film an der Innenwand eines Rundkolbens entsteht. Der Film wird mit 10 mL 0,1%igem Zitratpuffer pH 7,3 von der Gefäßwand abgeschwemmt und die so erhaltene Emulsion wie in Beispiel 1 weiterbehandelt und sterilfiltriert.
7. Virussichere Aktivatorsubstanzen zur Aktivierung des *intrinsic* Tenase-Pathways.
   Als Aktivatorsubstanz wird entweder Gerinnungsfaktor Xla allein, bzw. gemeinsam mit Gerinnungsfaktor XI verwendet oder Gerinnungsfaktor Vlla, vorzugsweise mit Tissue Faktor. Gerinnungsfaktor Xla kann auch gemeinsam mit Gerinnungsfaktor VIIa und Tissue Faktor angewandt werden. Zur Verwendung der aktivierten Gerinnungsfaktoren bzw. des Tissue Faktors als pharmazeutisch aktive Substanzen werden sie durch ein Solvent/Detergent-Verfahren virusinaktiviert und nach Entfernung des Solvent/Detergents einer Virusabreicherung durch Nanofiltration unterzogen.
   Nachstehende, pharmazeutisch aktive Substanzen, gelöst in einem 0,3%igen Zitratpuffer pH 7,3, werden als Stammlösungen der Aktivatorsubstanzen verwendet:
   a. 30 µg Faktor Xla /mL;
   b. 30 µg Faktor Xla und 50 ng Faktor XI/mL;
   c. 30 µg Faktor Xla und 20 ng Faktor VIIa/mL;
   d. 30 µg Faktor Xla und 20 ng Faktor VIIa und 10 ng Tissue Faktor/mL;
   e. 10 µg /mL Tissue Faktor
   Zur Auswahl der bestgeeigneten Aktivatorsubstanz werden je 10 µL einer Verdünnungsreihe der einzelnen Aktivatorsubstanzen zu je 1 mL Gerinnungsfaktorengemisch, das nach Beispiel 3 oder 4 erhalten wird, hinzugefügt und mit 10 µL einer 1:100 verdünnten Liposomenemulsion nach Beispiel 5 vermischt. Nach erfolgter Rekalzifizierung werden die Gemische bei 26° C bzw. 37° C gehalten. In Intervallen von 10, 30, 60 und 120 min wird mit Hilfe des chromogenen Substrats S-2251 die gebildete Menge Faktor Xa bestimmt. Es wird jene Aktivatorsubstanz zur Herstellung einer thrombingenerierfähigen, pharmazeutischen Wirkstoffzubereitung verwendet, die mit den nach Beispiel 3 oder 4 hergestellten Gerinnungsfaktorengemischen nach Rekalzifizierung die größte Menge Faktor Xa bildet.
8. Bestimmung der erforderlichen Mengen von Komponente C, die in einem Gemisch der Komponenten A und B innerhalb von 8 Stunden bei 26° C bzw. 2 Stunden bei 37° C mindestens 90% des vorhandenen Prothrombins in Thrombin umsetzen.
   Gerinnungsfaktorengemische nach Beispiel 3 oder 4, die erforderlichenfalls noch mit nach Beispiel 7 ermittelten Mengen von Aktivatorsubstanz versetzt sind, werden mit steigenden Mengen Liposomenemulsion vermischt. Zu je 10 mL eines Gerinnungsfaktorengemisches werden 0,1, 0,2, 0,4 und 0,8 mL einer 1:100 verdünnten, nach Beispiel 5 hergestellten Liposomenemulsion zugesetzt und nach Rekalzifizierung bei 26° C über 8 Stunden oder bei 37° C über 2 Stunden inkubiert. Es wird die geringste Menge Liposomenemulsion bestimmt, die erforderlich ist, um mindestens 90% des vorhandenen Prothrombins in Thrombin umzuwandeln.
9. Herstellung einer thrombingenerierfähigen, virussicheren pharmazeutischen Wirkstoffzubereitung.
   Ein nach Beispiel 3 hergestelltes, virussicheres Gerinnungsfaktorengemisch (Komponenten A und B) wird nach dem Auftauen, falls erforderlich, mit einer nach Beispiel 7 ausgewählten, virussicheren Aktivatorsubstanz in der entsprechenden Menge versetzt und das Gemisch bei 0° bis 2° C 15 Minuten lang gerührt. Zu diesem Gemisch wird die nach Beispiel 5 ermittelte Menge Liposomenemulsion (Komponente C) zugesetzt. Dieses Gemisch wird mit festem Kochsalz auf eine Konzentration von 0,9% NaCI gebracht, falls erforderlich, einer Klärfiltration unterzogen und dann durch ein Filter mit einer Porenweite von 0,22 µm und/oder 0,1 µm sterilfiltriert. Die gesamten Manipulationen, inklusive der Sterilfiltration, finden bei einer Temperatur zwischen 0° C und 2° C statt. Die so gewonnene, thrombingenerierfähige Wirkstoffzubereitung wird nach Probenziehung eingefroren und bei -20° C gelagert. Die erforderlichen Untersuchungen auf Stabilität und Pyrogenfreiheit werden nach der europäischen Pharmakopöe durchgeführt. In in-process Kontrollen und im sterilen Filtrat wird die thrombingenerierfähige Kapazität nach Rekalzifizierung bei 37° C über 2 Stunden bestimmt und ebenso noch vorhandenes, durch Ecarin aktivierbares Prothrombin, um die mindestens 90%ige Umwandlung des vorhandenen Prothrombins in Thrombin zu gewährleisten.
10. Herstellung einer thrombinhältigen, virussicheren, pharmazeutischen Wirkstoffzubereitung.
   Das nach Beispiel 4 aus einem Faktor-XIa-hältigen Prothrombinkomplexkonzentrat und einem Pro-Kofaktorenkonzentrat hergestellte, eingefrorene Gemisch wird aufgetaut und, wenn nach Beispiel 7 noch erforderlich, mit einer ausgewählten, berechneten Menge Aktivatorsubstanz versetzt. Durch Zugabe von 10 mg Tween 80 und 0,3 mg Trinitrobutylphosphat pro g Protein des Gerinnungsfaktorengemisches bei 26° C nach erfolgter Rekalzifizierung und Zusatz einer nach Beispiel 6 ermittelten Menge Liposomenemulsion wird in acht Stunden die Hauptmenge des Prothrombins in Thrombin übergeführt und gleichzeitig die erforderliche Virusinaktivierung durchgeführt.
   Die thrombinhältige Lösung kann entweder eingefroren und gelagert oder sofort bzw. nach Wiederauftauen weiterverarbeitet werden. Durch Zusatz einer entsprechenden Natriumzitratlösung wird eine 25 mMolarität und ein pH-Wert von 6,5 eingestellt und unter weiterem Rühren bei Zimmertemperatur soviel Polyäthylenglykol mit einem Molekulargewicht zwischen 6.000 und 8.000 zugesetzt, bis eine 0,1%ige Lösung entsteht. Nunmehr wird eine mit 0,025 M Natriumzitrat pH 6,5, gewaschene, 20%ige CM-Sepharose-Suspension unter Rühren zugesetzt, bis mindestens 95% des vorhandenen Thrombins an die CM-Sepharose adsorbiert sind. Die erforderliche Menge CM-Sepharose wird in einem Vorversuch bestimmt, indem die geringste Menge der 20%igen Emulsion ermittelt wird, die mindestens 95% des vorhandenen Thrombins adsorbiert. Die CM-Sepharose wird während 30 Minuten zwischen 3.000 bis 5.000 g abzentrifugiert, das Sediment in 25 mM Zitratpuffer pH 6,5 resuspendiert und in eine entsprechende Kolonne eingefüllt. Die Kolonne wird mit 1 %igem Zitratpuffer, der noch 0,1% PEG enthält, bei pH 6,5 gewaschen und mit einem Kochsalzgradienten in Anwesenheit von 0,1% Zitrat und 0,1% PEG bei pH 6,5 eluiert. Der Kochsalzgradient wird zwischen 10 und 200 mM errichtet. Die Fraktionen, die das meiste Thrombin enthalten, werden gesammelt, diafiltriert und dadurch auf eine Konzentration von etwa 500 E Thrombin pro mL gebracht. Eine solche Lösung kann eingefroren, nach Gefriertrocknung gelagert oder sofort weiterverarbeitet werden.
   Nach vorheriger Klärfiltration durch Filter verschiedener Porengröße zwischen 5.000 und 75 nm wird die thrombinhältige Lösung durch einen Nanofilter mit 35 nm Porengröße und vorzugsweise durch weitere Nanofilter mit Porendurchmessern von 20 und 15 nm filtriert. Die so erhaltene virusabgereicherte Thrombinlösung wird durch Ultrafiltration mit Hilfe eines 30 KD Filters auf einen Gehalt von über 5.000 E Thrombin pro mL konzentriert und eingefroren oder gefriergetrocknet gelagert.
11. Herstellung eines thrombingenerierfähigen virussicheren Arzneimittels.
   Eine äquivalente Probe des nach Beispiel 9 hergestellten, eingefrorenen oder gefriergetrockneten, virussicheren, pharmazeutischen Wirkstoffes wird mit einer 0,9%igen NaCI-Lösung so verdünnt, dass die Probe nach Rekalzifizierung bei 37° C innerhalb von 2 Stunden 500 ± 50 Einheiten Thrombin pro mL generiert.
   Eine solche verdünnte Probe wird mit gleichen Teilen eines 5 - 10%igen, fibrinogenhältigen Arzneimittels, das als Bestandteil eines Fibrinkleberkits vorgesehen ist, vermischt und die Gerinnungszeit dieser Mischung nach Rekalzifizierung bestimmt. Wenn die Gerinnungszeit bei 37° C länger als 150 Sekunden ist, wird der Wirkstoffzubereitung nach Beispiel 7 noch soviel nach Beispiel 8 .hergestelltes Thrombin zugesetzt, bis die Gerinnungszeit auf zwischen 100 und 150 Sekunden gesunken ist.
   Die so ermittelten Mengen Thrombin werden dem nach Beispiel 9 erhaltenen Wirkstoff unter Rühren bei einer Temperatur von zwischen 0° C und 2° C zugesetzt und diese Mischung nach Klärfiltration sterilfiltriert. Die erhaltene, sterile Bulklösung wird vorzugsweise bei derselben niederen Temperatur in Endverbraucherfläschchen portioniert, verfüllt und gefriergetrocknet. Die Auflösung des gefriergetrockneten Pulvers erfolgt mit einer sterilen 5 mM CaCl₂-Lösung.
12. Herstellung eines thrombinhältigen, virussicheren Arzneimittels.
   Eine Wirkstoffzubereitung nach Beispiel 10 wird nach Auftauen oder Auflösen mit Kochsalz auf eine Konzentration von 0,9% NaCI gebracht und dann durch Zugabe einer isotonen Kochsalzlösung die gewünschte Thrombinkonzentration auf zwischen 500 und 5.000 Einheiten pro mL eingestellt. Es wird soviel PEG zugesetzt, dass eine 0,1%ige Lösung entsteht und mit Hilfe einer 10%igen CaCl₂-Lösung eine 5 mM CaCl₂-Lösung erhalten wird. Diese Lösung wird sterilfiltriert, portioniert in Endverbrauchergefäße steril abgefüllt und kann eingefroren oder nach Gefriertrocknung gelagert werden. Die Rekonstitution der gefriergetrockneten Thrombinlösung erfolgt mit WFI.
13.Anwendung eines thrombingenerierfähigen Arzneimittels gemeinsam mit einem thrombinhältigen Arzneimittel.
   1 mL eines fibrinogenhältigen Arzneimittels, das 50 - 100 mg Fibrinogen pro mL enthält, wird mit 1 mL eines thrombingenerierfähigen Arzneimittels nach Beispiel 11 vermischt. Die Mischung dieser beiden Arzneimittel wird unter sterilen Bedingungen etwa 20 Sekunden lang stark gerührt und die so erhaltene homogene Mischung auf die zu klebende bzw. abzudichtende Stelle aufgebracht, nachdem die zu verklebenden oder abzudichtenden Gewebeteile adaptiert wurden, und so lange in der adaptierten Position gehalten, bis das Gemisch beider Arzneimittel geronnen ist. Falls erforderlich, können die Gewebeteile auch noch längere Zeit in einer fixierten Position verbleiben, die bis zu mehreren Stunden betragen kann.
14.Anwendung eines thrombinhältigen Arzneimittels.
   Zu 2,0 mL eines erfindungsgemäßen Arzneimittelgemisches nach Beispiel 13 werden 0,2 mL eines thrombinhältigen Arzneimittels nach Beispiel 12 mit 2.000 Einheiten Thrombin pro mL zugesetzt und rasch vermischt. Dieses Gemisch wird zur Blutstillung rasch auf blutende Stellen aufgebracht, möglichst nach Entfernung von angesammeltem Blut, entweder durch Absaugen bzw. Aufsaugen oder durch Verblasen des Blutes.
15. Bestimmung des Gerinnungsfaktors XI und des Gerinnungsfaktors XII in Ionenaustauscher-Eluaten nach Beispiel 4a, die nicht nach ihrer Beladung gelagert, sondern nach dem Waschen des beladenen Ionenaustauschers sofort eluiert werden. Die Bestimmung erfolgt mit Hilfe der entsprechenden Gerinnungsfaktoren-Mangelplasmen von American Diagnostica.
16. Bestimmung von Kallikrein und Präkallikrein in Eluaten von Ionenaustauschern, die mit Prothrombinkomplexen beladen und nach dem Waschen sofort oder erst nach Lagerung bis zu 100 Stunden bei Kühlschranktemperatur eluiert wurden. Die Kallikreinaktivität wird mit Hilfe des chromogenen Substrats S-2403 bestimmt, wobei zur Bestimmung eine 4 mM chromogene Substratlösung verwendet wird, die im Gesamtansatz in einer 0,4 mM Konzentration vorliegt. Die Präkallikreinkonzentration wird ebenfalls mit chromogenem Substrat S-2403 bestimmt, indem das Eluat in einer 1:10 Verdünnung mit gleichem Volumen einer 0,5%igen Kaolinsuspension versetzt und 5 Minuten bei 37° C unter Schütteln inkubiert wird. Von der gemessenen Extinktion wird die Extinktion, die ohne Aktivierung mit Kaolinsuspension erhalten wird, abgezogen und auf einer Standardkurve die Mengen, bzw. Einheiten Präkallikrein ermittelt.
17. Bestimmung von Präkallikrein-Aktivator. Die Bestimmung erfolgt gleichartig wie die Bestimmung von Präkallikrein, nur dass anstelle von Kaolinsuspensionen eine Konzentrationsreihe von Präkallikrein-Aktivatoren hergestellt wird. Wenn Kupfersalze der Ellagsäure verwendet werden, wird eine Konzentrationsreihe von zwischen 10 ng bis 10000 ng verwendet, die aus einer vorgelegten Menge Präkallikrein innerhalb von 5 Minuten bei 37° C die maximale Menge Kallikrein bildet.
18. Herstellung pflanzlicher und/oder synthetischer Phospholipid-Emulsionen aus L-α-Phosphatidylcholin, L-α-Phosphatidyl-L-Serin und L-α-Phosphatidyläthanolamin, die in gleicher Form erfolgt wie sie in Beispiel 5 angegeben ist.
19. Bestimmung von TAFIa. Die Bestimmung von TAFIa erfolgt mit Hilfe eines Chromogenic TAFI Activity Kits von American Diagnostika ohne Zusatz von Aktivator (Thrombin). Der Gehalt an TAFIa wird aus einer Standardkurve errechnet, die mit Verdünnungen von Plasma und nachfolgender Aktivierung mit Thrombin und Messung der TAFIa-Aktivität mit Hilfe eines chromogenen Substrats ermittelt wurde.
20. Bestimmung von Gerinnungsfaktor XIIIa. Die Bestimmung von aktiviertem Faktor XII erfolgt nach der European Pharmacopoeia Suppl. 4.5. 07/2003, p. 3687 ohne Verwendung von Aktivatoren des Faktors XIII (Thrombin).
21. Ultraschallbehandlung von Phospholipid-Emulsionen. Es werden durch Verdünnung mit isotoner Kochsalzlösung aus den Phospholipid-Gemischen 1%ige Suspensionen hergestellt und 2 mL einer Beschallung unterzogen. Die Beschallung wird mit dem Ultraschall-Desintegrator Sonifire II W-250 durchgeführt. Die Ausgangsleistung beträgt maximal 200 Watt bei einer Arbeitsfrequenz von 20 kHz. Verwendet wurde Konverter 102 C mit Standard-Resonator 1/2" mit Mikrospitze 101-148-062. Die Beschallungsdauer wird zwischen 10 und 100 Sekunden ohne Kühlung der beschallten Emulsion bei Stufe 1 und bei 10% Pulsierintervall durchgeführt. Die Aktivität der beschallten Phospholipid-Emulsion wird mit Hilfe eines Referenzplasmas und einer Kaolinsuspension durch Bestimmung der partiellen Thromboplastinzeit durchgeführt. 100 µL Plasma werden mit 50 µL einer Liposomenemulsion und 50 µL einer 0,5%igen Kaolinsusension 5 Minuten bei 37° C inkubiert, rekalzifiziert, und die Gerinnungszeit bei 37° C bestimmt. Es wird die erforderliche Menge Liposomenemulsion bestimmt, die die kürzeste Gerinnungszeit verursacht. Die Wirksamkeit der in Beispiel 5 und 6 angeführten Liposomen-Emulsionen kann ebenso wie die in Beispiel 18 angeführten Phospholipid-Emulsionen durch diese Methode bestimmt werden.
22. Ermittlung der geeigneten Ca-Ionenkonzentration zur erwünschten Einstellung der Gerinnungszeit einer thrombingenerierungsfähigen, pharmazeutischen Wirkstoffzubereitung. Thrombingenerierende, pharmazeutische Zubereitungen werden hergestellt, und die zu untersuchende pharmazeutische Zubereitung wird in einer geometrischen Verdünnungsreihe mit Ca-Ionen versetzt, in der die höchste Ca-Ionenkonzentration 1 mM ist. Die Verdünnungsreihe wird von 125 µm bis 1000 µm angesetzt. In gleichartiger Weise wird eine Ca-Ionenkonzentrationsreihe von 10 mM bis 640 mM angesetzt und die Gerinnungszeit bestimmt. Die erforderliche Ca-Ionenkonzentration wird mit Hilfe einer Standardkurve ermittelt.

### Literatur

Blombäck B. Fibrinogen: Evolution of the Structure-Function Concept: Keynote Address at Fibrinogen 2000 Congress. Annals N.Y. Acad. Sci. 2001; 936:1-10
Booth N.A. TAFI Meets the Sticky Ends. Thromb. Haemost. 2001; 85:1-2
Davie E.W., Ratnoff O.D. Waterfall sequence for intrinsic blood clotting. Science 1964; 145:1310-12
Drake T.A., Morrissey J.H., Edgington T.S. Selective cellular expression of tissue factor in human tissue. Implication for disorders of hemostasis and thrombosis. Am. J. Path. 1989; 134:1087-97
Grey E.G. Fibrin as a haemostatic in cerebral surgery. Surg. Gyn. Obst. 1915; 21:452-454
MacFarlane R.G. An enzyme cascade in the blood clotting mechanism and its function as an biochemical amplifier. Nature 1964; 202:498-9
Mann K.G., Jenny R.J., Krishnaswamy. Cofactor proteins in the assembly and expression of blood clotting enzyme complexes. Ann. Rev. Biochem. 1988; 57:915-56
Matras H. et al. Zur Klebung von Nervenanastomosen mit Gerinnungssubstanzen. Fortschr. Kiefer-Gesichts-Chir. 1976; 112-114.
Morawitz P. Die Chemie der Blutgerinnung. Ergebn. d. Physiol. 1905; 4:307
Rapaport S.I., Rao L.V.M. The Tissue Factor Pathway: How it has become a "Prima Ballerina". Thromb. Haemost. 1995; 74:7-17
Siebenlist K.R., Meh D.A., Mosesson M.W. Protransglutaminase (F-XIII) mediated cross-linking of fibrinogen and fibrin. Thromb. Haemost. 2001; 86:1221-8
Spängler H.P. Gewebeklebung und lokale Blutstillung mit Fibrinogen, Thrombin und Blutgerinnungsfaktor XIII (Experimentelle Untersuchungen und klinische Erfahrungen). Wien. klin. Wschr. 1976; 88(4):3-18
von dem Borne P.A.K., Koppelman S.J., Bouma B.N. et al. Surface independent factor XI activation by thrombin in the presence of high molecular weight kininogen. Thromb. Haemost. 1994; 72:397-402
Young F. et al. "Suture" of Wounds by Plasma-Thrombin Adhesion. War Med. 1944; 6:80-85

## Patentansprüche

1. Pharmazeutische Wirkstoffzubereitung zur Herstellung eines thrombingenerierfähigen oder thrombinhältigen Arzneimittels, **dadurch gekennzeichnet, dass** sie enthält:
(A) aus Plasma oder gentechnologisch gewonnenes Prothrombin (Gerinnungsfaktor II),
(B) aus Plasma oder gentechnologisch gewonnene Gerinnungsfaktoren V, VIII, IX, X, die zumindest teilweise in aktiviertem Zustand vorliegen können, und aus Plasma oder gentechnologisch gewonnenen Gerinnungsfaktor Xla, und
(C)prionensichere, gerinnungsaktive Phospholipide, wobei die Phospholipide gegebenenfalls in Liposomen enthalten sind.

2. Pharmazeutische Wirkstoffzubereitung nach Anspruch 1, **dadurch gekennzeichnet, dass** sie auch aus Plasma oder gentechnologisch gewonnenen Gerinnungsfaktor VII oder ein Gemisch von aus Plasma oder gentechnologisch gewonnenen Gerinnungsfaktoren VII und Vlla enthält.

3. Pharmazeutische Wirkstoffzubereitung nach einem der Ansprüche 1 oder 2,
**dadurch gekennzeichnet, dass** sie einen gentechnologisch hergestellten Tissue Faktor als solchen oder in relipidierter Form enthält.

4. Pharmazeutische Wirkstoffzubereitung nach einem der Ansprüche 1 bis 3,
**dadurch gekennzeichnet, dass** die Gerinnungsfaktoren ausschließlich aus Blutplasma einer bestimmten Säugetierspezies hergestellt sind oder aus den entsprechenden gentechnologisch gewonnenen Gerinnungsfaktoren oder aktivierten Gerinnungsfaktoren, einschließlich Tissue Faktor, hergestellt sind.

5. Pharmazeutische Wirkstoffzubereitung nach einem der Ansprüche 1 bis 4,
**dadurch gekennzeichnet, dass** das Prothrombin, die Gerinnungsfaktoren V, VIII, IX, X, Xla und gegebenenfalls die Gerinnungsfaktoren VII und VIIa, sowie der Tissue Faktor durch Virusinaktivierung und/oder Virusabreicherung virussicher sind.

6. Pharmazeutische Wirkstoffzubereitung nach einem der Ansprüche 1 bis 5,
**dadurch gekennzeichnet, dass** sie in tiefgefrorener oder gefriergetrockneter Form vorliegt.

7. Thrombingenerierfähiges Arzneimittel, herstellbar aus einer pharmazeutischen Wirkstoffzubereitung nach einem der Ansprüche 1 bis 5.

8. Thrombingenerierfähiges Arzneimittel nach Anspruch 7, **dadurch gekennzeichnet, dass** es in tiefgefrorenem oder in gefriergetrocknetem Zustand vorliegt.

9. Thrombingenerierfähiges Arzneimittel nach Anspruch 7, **dadurch gekennzeichnet,**
**dass** es zusätzlich Thrombin in einer solchen Menge enthält, dass es nach Auftauen oder Rekonstitution nicht mehr als 1 Einheit Thrombin pro ml enthält.

10. Thrombinhältiges Arzneimittel, herstellbar aus einer thrombingenerierfähigen, pharmazeutischen Wirkstoffzubereitung nach einem der Ansprüche 1 bis 6.

11. Thrombinhältiges Arzneimittel nach Anspruch 10, **dadurch gekennzeichnet, dass** es in tiefgefrorenem oder gefriergetrockneten Zustand vorliegt.

12. Arzneimittelgemisch, das vor seiner Anwendung durch Vermischen eines Arzneimittels nach einem der Ansprüche 7 bis 9 mit einem fibrinogenhältigen Arzneimittel innerhalb von 30 bis 300 Sekunden gerinnt.

13. Thrombinhältiges Arzneimittel nach einem der Ansprüche 10 oder 11, das mit einem Arzneimittelgemisch nach Anspruch 12 versetzt, innerhalb von 3 bis 10 Sekunden gerinnt.

14. Verfahren zur Herstellung einer thrombinhältigen pharmazeutischen Wirkstoffzubereitung, **dadurch gekennzeichnet, dass** eine thrombingenerierfähige Wirkstoffzubereitung mit Ca-Ionen versetzt wird, wobei ein thrombinhältiges Produkt gebildet wird, welches anschließend chromatographisch gereinigt wird.

15. Verfahren nach Anspruch 14, **dadurch gekennzeichnet, dass** die Wirkstoffzubereitung durch Virusinaktivierung während oder nach der Thrombinbildung, sowie durch Virusabreicherung nach abgeschlossener Thrombinbildung virussicher gemacht wird.

16. Verfahren zur Herstellung eines Pro-Kofaktorenkonzentrates enthaltend die Gerinnungsfaktoren V und VIII, **dadurch gekennzeichnet, dass** aus Plasma gewonnenes Kryopräzipitat gelöst und aus der Lösung Fibrinogen abgetrennt wird, wobei eine die Gerinnungsfaktoren V und VIII hältige Lösung erhalten wird, welche mit Calciumchlorid versetzt werden kann.

17. Verfahren zur Herstellung eines Gerinnungsfaktor-XI-hältigen Prothrombinkomplexkonzentrates enthaltend die Gerinnungsfaktoren II, VII, VIIa, IX, X und XI, wobei pro 1,0 µg Prothrombin mindestens 0,030 µg Gerinnungsfaktor XIa enthalten sind, **dadurch gekennzeichnet, dass** aus Plasma gewonnener Kryopräzipitatüberstand an einem Anionenaustauscher adsorbiert und nach Waschen des Adsorbats und Lagerung in der Kälte Gerinnungsfaktor-XIa-hältiger Prothrombinkomplex eluiert wird.

18. Pharmazeutische Wirkstoffzubereitungen nach den Ansprüchen 1-6 aus virusinaktivierten, thrombin-generierenden, Gerinnungsfaktor-XI- und Gerinnungsfaktor-XII-hältigen Prothrombinkomplexen, die vorzugsweise in Anwesenheit gerinnungsaktiver Phospholipide durch Zusatz von virusinaktiviertem oder von virussicherem Kallikrein oder virusinaktiviertem bzw. virussicherem Präkallikrein und mit virussicheren Präkallikreinaktivatoren aktiviert werden.

19. Pharmazeutische Wirkstoffzubereitungen nach den Ansprüchen 1-6 und 18, die außer Prothrombinkomplexen auch noch TAFI oder TAFI und Gerinnungsfaktor XIII enthalten und nach der Aktivierung von Prothrombin einen Gehalt von mindestens 3 E (50 µg) TAFIa bzw. 3 E TAFIa und 5 E Gerinnungsfaktor XIIIa pro 1000 E Thrombin aufweisen.

20. Pharmazeutische Wirkstoffzubereitungen nach den Ansprüchen 1 - 6 und Zubereitungen von Arzneimittelgemischen nach den Ansprüchen 7 - 13, 18 und 19, **dadurch gekennzeichnet, dass** prionensichere, gerinnungsaktive Phospholipide pflanzlichen Ursprungs oder solche pflanzlichen und synthetischen Ursprungs verwendet werden, vorzugsweise in einem Verhältnis von Phosphatidylcholinen zu Phosphatidylserinen von 3+1 oder von Phosphatidylcholinen zu Phosphatidylserinen zu Phosphaäthanolaminen von 2 + 2 + 1.

21. Herstellung von pharmazeutischen Wirkstoffzubereitungen nach den Ansprüchen 1 - 6, 18, 19 und 20 sowie von Arzneimitteln nach Ansprüchen 7-13, **dadurch gekennzeichnet, dass** die zu verwendenden Phospholipidemulsionen noch einer Ultraschallbehandlung und daraffolgend allenfalls einer Sterilfiltration unterzogen werden.

22. Herstellung von thrombingenerierfähigen, virussicheren Arzneimitteln nach Anspruch 9 und pharmazeutischen Wirkstoffzubereitungen nach Ansprüchen 18 und 19, wobei als Komponente C nach Ansprüchen 20 und 21 hergestellte Phospholipidemulsionen verwendet werden.

23. Herstellung von thrombinhältigen, virussicheren Arzneimitteln nach Anspruch 10 und pharmazeutische Wirkstoffzubereitungen nach den Ansprüchen 18 oder 19, wobei als Komponente C nach Ansprüchen 20 und 21 hergestellte Phospholipidemulsionen verwendet werden.

24. Verfahren zur Herstellung von Arzneimitteln nach den Ansprüchen 7-9, 22, sowie eines Arzneimittelgemisches nach Anspruch 12, indem eine Ca-lonenkonzentration unter 1 mM oder über 10 mM zur Anwendung kommt.
